# EUROPEAN PATENT SPECIFICATION

(11) **EP 3 352 734 B1**
(45) Date of publication and mention of the grant of the patent: **15.01.2020**
(21) Application number: 16775134.6
(22) Date of filing: 22.09.2016
(51) Int. Cl.: A61K 9/06, A61K 31/519, A61P 17/14, A61K 47/06, A61K 9/00

(54) **TREATMENT OF ALOPECIA AREATA**
BEHANDLUNG VON ALOPECIA AREATA
TRAITEMENT DE LA PELADE

(30) Priority: 24.09.2015 EP 15186644; 29.09.2015 JP 2015190849
(43) Date of publication of application: 01.08.2018
(73) Proprietor: Leo Pharma A/S, 2750 Ballerup (DK); JAPAN TOBACCO INC., Minato-ku Tokyo 105-8422 (JP)
(72) Inventor: SIERRA, Michael, 2750 Ballerup (DK); LABUDA, Tord, 2750 Ballerup (DK); TANIMOTO, Atsuo, Tokyo 105-8422 (JP); SHINOZAKI, Yuichi, Tokyo 105-8422 (JP)
(74) Representative: Leo Pharma A/S
(86) International application number: PCT/EP2016/072531
(87) International publication number: WO 2017/050891

(56) References cited:
- WO-A1-2015/060208

## Description

### FIELD OF THE INVENTION

The present invention relates to a new pharmaceutical use of 3-[(3S,4R)-3-methyl-6-(7H-pyrrolo[2,3-d]pyrimidin-4-yl)-1,6-diazaspiro[3.4]octan-1-yl]-3-oxopropanenitrile. More specifically, the present invention relates to a therapeutic or preventive agent for alopecia areata which contains 3-[(3S,4R)-3-methyl-6-(7H-pyrrolo[2,3-d]pyrimidin-4-yl)-1,6-diazaspiro[3.4]octan-1-yl]-3-oxopropanenitrile.

### BACKGROUND OF THE INVENTION

Alopecia areata (AA) is an autoimmune disease that results in hair loss that ranges in presentation from circular patches on the scalp that can often undergo spontaneous resolution to complete hair loss that may persist for life. There are currently no FDA-approved treatments for AA, and treatment regimens for patients with severe disease are empiric and frequently unsatisfactory.

### CITATION LISTS

### Patent literature:

Patent literature 1: JP 2011-46700 A (Japan Tobacco Inc.)
Patent literature 2: WO2011/013785 (Japan Tobacco Inc.)

### Non-patent literature:

Non-patent literature 1: MCELWEE, KJ et al. Experimental induction of alopecia areata-like hair loss in C3H/HeJ mice using full-thickness skin grafts. J Invest Dermatol. Nov 1998, Vol. 111, No. 5, pages 797-803.

### SUMMARY OF THE INVENTION

The invention is defined by the claims. Any subject-matter falling outside the scope of the claims is provided for information purposes only. Any references in the description to methods of treatment (and uses of claimed compounds) refer to the compounds, pharmaceutical compositions and medicaments of the present invention for use in a method for treatment of the human or animal body by therapy.

### (A):

The problem to be solved by the invention is to provide a new pharmaceutical use of 3-[(3S,4R)-3-methyl-6-(7H-pyrrolo[2,3-d]pyrimidin-4-yl)-1,6-diazaspiro[3.4]octan-1-yl]-3-oxopropanenitrile.

The present inventors found for the first time that 3-[(3S,4R)-3-methyl-6-(7H-pyrrolo[2,3-d]pyrimidin-4-yl)-1,6-diazaspiro[3.4]octan-1-yl]-3-oxopropanenitrile is effective for the treatment or prevention of alopecia areata and that 3-[(3S,4R)-3-methyl-6-(7H-pyrrolo[2,3-d]pyrimidin-4-yl)-1,6-diazaspiro[3.4]octan-1-yl]-3-oxopropanenitrile is effective as a topical agent for treating alopecia areata or a topical agent for preventing alopecia areata, and achieved the present invention.

Specifically, the present invention includes the following aspects.
[1] A therapeutic or preventive agent for use in treating alopecia areata, comprising a compound represented by the following chemical structural formula: 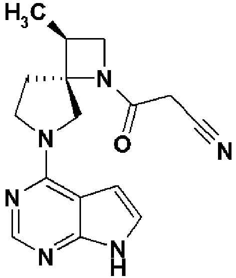 or a pharmaceutically acceptable salt thereof as an active ingredient.
[2] The therapeutic or preventive agent for use of [1], wherein the therapeutic or preventive agent for alopecia areata is a topical agent.
[3] The therapeutic or preventive agent for use of [2], wherein the topical agent is an ointment.
[4] The therapeutic or preventive agent for use of [3], wherein the concentration of 3-[(3S,4R)-3-methyl-6-(7H-pyrrolo[2,3-d]pyrimidin-4-yl)-1,6-diazaspiro[3.4]octan-1-yl]-3-oxopropanenitrile in the ointment of [3] is 0.3% by weight.
[5] The therapeutic or preventive agent for use of [3], wherein the concentration of 3-[(3S,4R)-3-methyl-6-(7H-pyrrolo[2,3-d]pyrimidin-4-yl)-1,6-diazaspiro[3.4]octan-1-yl]-3-oxopropanenitrile in the ointment of [3] is 1% by weight.
[6] The therapeutic or preventive agent for use of [3], wherein the concentration of 3-[(3S,4R)-3-methyl-6-(7H-pyrrolo[2,3-d]pyrimidin-4-yl)-1,6-diazaspiro[3.4]octan-1-yl]-3-oxopropanenitrile in the ointment of [3] is 3% by weight.

Also disclosed is a new pharmaceutical use of 3-[(3S,4R)-3-methyl-6-(7H-pyrrolo[2,3-d]pyrimidin-4-yl)-1,6-diazaspiro[3.4]octan-1-yl]-3-oxopropanenitrile.

Also disclosed is the use of 3-[(3S,4R)-3-methyl-6-(7H-pyrrolo[2,3-d]pyrimidin-4-yl)-1,6-diazaspiro[3,4]octan-1-yl]-3-oxopropanenitrile in the treatment of alopecia areata.

Also disclosed is the above use administered as a twice daily application.

Also disclosed is the above use administered in a concentration of 30 mg/g.

Also disclosed is the above use administered in a concentration of 10 mg/g.

Also disclosed is the above use administered in a concentration of 5 mg/g.

### BRIEF DESCRIPTION OF THE DRAWING

Fig. 1 A chart illustrating the promotion of hair growth by Compound A in a C3H/HeJ mouse alopecia model. (Example 1)
Fig. 2 A chart illustrating the promotion of hair growth by Compound A in a C3H/HeJ mouse alopecia model. (Example 2)
Terms and phrases used herein are defined as below:

A compound represented by the following chemical structural formula: 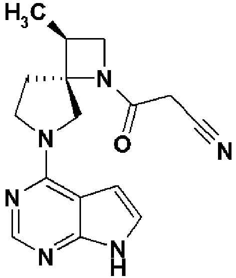 is called Compound A.

The chemical name of Compound A is 3-[(3S,4R)-3-methyl-6-(7H-pyrrolo[2,3-d]pyrimidin-4-yl)-1,6-diazaspiro[3.4]octan-1-yl]-3-oxopropanenitrile.

Compound A can be produced according to the method described in Preparation 6 of Patent Literature 1 and 2.

Compound A is known to inhibit JAK.

The "pharmaceutically acceptable salt" may be any non-toxic salts of Compound A, and includes a salt with an inorganic acid, a salt with an organic acid, a salt with an inorganic base, a salt with an organic base, and a salt with an amino acid, etc.

The salt with an inorganic acid includes a salt with hydrochloric acid, nitric acid, sulphuric acid,hosphoriccid, hydrobromic acid, etc.

The salt with anorganic acid includes salt with oxalic acid, maleic acid, citric acid, fumaric acid, lactic acid, malic acid, succinic acid, tartaric acid, acetic acid, trifluoroacetic acid, gluconic acid, ascorbic acid, methanesulfonic acid, benzenesulfonicacid,p-toluenesulfonic acid, etc.

The salt with an inorganic base includes sodium salt, potassium salt, calcium salt, magnesium salt, ammonium salt, etc.

The salt with an organic base includes a salt with methylamine, diethylamine, trimethylamine, triethylamine, ethanolamine, diethanolamine, triethanolamine, ethylenediamine, tris(hydroxymethyl)methylamine, dicyclohexylamine, N,N'-dibenzylethylenediamine, guanidine, pyridine, picoline, choline, cinchonine, meglumine, etc.

The salt with an amino acid includes a salt with lysine, arginine, aspartic acid, glutamic acid, etc.

According to well-known methods, each salt may be obtained by reacting Compound A with an inorganic base, an organic base, an inorganic acid, an organic acid or an amino acid.

Compound A may be labeled with isotopes (e.g., ¹⁴C, ³⁵S, etc.).

Preferable, Compound A or a pharmaceutically acceptable salt thereof is substantially purified Compound A or a pharmaceutically acceptable salt thereof. A more preferable one is Compound A or a pharmaceutically acceptable salt thereof which is purified in the purity of 80% or more.

The "pharmaceutical composition" includes an oral preparation such as tablet, capsule, granule, powder, lozenge, syrup, emulsion and suspension, or a parenteral preparation such as topical agent, suppository, injection, eyedrop, nasal drug and pulmonary drug. A preferable example of the "pharmaceutical composition" is a topical agent. A more preferable example of the "pharmaceutical composition" is an ointment.

The pharmaceutical composition of the present invention is produced by appropriately mixing Compound A or a pharmaceutically acceptable salt thereof with at least one type of pharmaceutically acceptable carriers in appropriate amounts accordingto methods known in the technical field of medicinal preparation. Thecontentof Compound A or a pharmaceutically acceptable salt thereof in the pharmaceutical composition depends on its dosage forms, dosage amounts, etc., and for example, is 0.1 to 100% by weight of the entire composition. For example, the content is 0.25, 0.30,0.50, 0.75, 1.0,1.25,1.50, 1.75, 2.00, 2.25, 2.50, 2.75, 3.00, 3.25, 3.50, 3.75 or 4.00% by weight of the entire composition. A preferable content of Compound A or a pharmaceutically acceptable salt thereof in the pharmaceutical composition is 0.3% by weight, 0.5% by weight, 1% by weight or 3% by weight of the entire composition. In this regard, each numerical value may be increased or decreased by an increment of 0.25. Preferably, each numeral value may be increased or decreased by an increment of 0.1.

The term "effective amount" as used herein means a dosage which is sufficient in order for the treatment of the patient to be effective compared with no treatment.

The term "therapeutically effective amount" of the compound as used herein means an amount sufficient to cure, alleviate or partially arrest the clinical manifestations of a given disease and its complications. An amount adequate to accomplish this is defined as "therapeutically effective amount". Effective amounts for each purpose will depend on the severity of the disease or injury as well as the weight and general state of the subject.

The "pharmaceutically acceptable carrier" includes various conventional organic or inorganic carrier substances for pharmaceutical materials, e.g., an excipient, a disintegrant, a binder, a fluidizer, and a lubricant for solid preparations, a solvent, a solubilizing agent, a suspending agent, a tonicity agent, a buffer, and a soothing agent for liquid preparations, and a base, an emulsifier, a humectant, astabilizer,astabilizing agent, a dispersant, a plasticizer, a pH regulator, an absorption promoter, a gelling agent, an antiseptic, a filler, a resolvent, a solubilizing agent, and a suspending agent for semisolid preparations. Further, an additive including a preserving agent, an antioxidant agent, a colorant, and a sweetening agent may be used, if needed.

The "excipient" includes lactose, white soft sugar, D-mannitol, D-sorbitol, cornstarch, dextrin, microcrystalline cellulose, crystalline cellulose, carmellose, carmellose calcium, sodium carboxymethyl starch, low-substituted hydroxypropyl cellulose, gum arabic, etc.

The "disintegrant" includes carmellose, carmellose calcium, carmellose sodium, sodium carboxymethyl starch, croscarmellose sodium, crospovidone, low-substituted hydroxypropyl cellulose, hydroxypropyl methylcellulose, crystalline cellulose, etc.

The "binder" includes hydroxypropyl cellulose, hydroxypropyl methylcellulose, povidone, crystalline cellulose, white soft sugar, dextrin, starch, gelatin, carmellose sodium, gum arabic, etc.

The "fluidizer" includes light anhydrous silicic acid, magnesium stearate, etc.

The "lubricant" includes magnesium stearate, calcium stearate, talc, etc.

The "solvent" includes purified water, ethanol, propylene glycol, macrogol, sesame oil, corn oil, olive oil, etc.

The "solubilizing agent" includes propylene glycol, D-mannitol, benzylbenzoate, ethanol, triethanolamine, sodium carbonate, sodium citrate, etc.

The "suspending agent" includes benzalkonium chloride, carmellose, hydroxypropyl cellulose, propylene glycol, povidone, methyl cellulose, glyceryl monostearate, etc.

The "tonicity agent" includes glucose, D-sorbitol, sodium chloride, D-mannitol, etc.

The "buffer" includes sodium hydrogen phosphate, sodium acetate, sodium carbonate, sodium citrate, etc.

The "soothing agent" includes benzyl alcohol, etc.

The "base" includes water, animal or plant oils (e.g., olive oil, corn oil, peanut oil, sesame oil, castor oil, squalane, etc.), lower alcohols (e.g., ethanol, propanol, propylene glycol, 1,3-butylene glycol, phenol, etc.), higher fatty acids and esters thereof, waxes, higher alcohols, polyhydricalcohols, hydrocarbons (e.g., whitesoft paraffin, liquid paraffin, paraffin, hard paraffin, etc.), hydrophilic petrolatum, purified lanolin, absorptive ointment, hydrous lanolin, hydrophilic ointment, starch, pullulan, gum arabic, tragacanth gum, gelatin, dextran, cellulose derivatives (e.g., methyl cellulose, carboxymethyl cellulose, hydroxyethyl (e.g., carboxyvinyl polymer, sodium polyacrylate, polyvinyl alcohol, polyvinyl pyrrolidone, etc.), propylene glycol, macrogol (e.g., macrogol 200 to 600, etc.), and combinations of two or more kinds of these. Preferably, the base is a combination of white soft paraffin, hard paraffin and squalane.

The "preserving agent" includes ethyl parahydroxybenzoate, chlorobutanol, benzyl alcohol, sodium dehydroacetate, sorbic acid, etc.

The "antioxidant agent" includes sodium sulfite, ascorbic acid, etc.

The "colorant" includes food dye (e.g., Food Red No. 2 or No. 3, Food Yellow No. 4 or No. 5, etc.), β-carotene, etc.

The "sweetening agent" includes saccharin sodium, dipotassium glycyrrhizinate, aspartame, etc.

The carrier is preferably a combination of white soft paraffin, hard paraffin and squalane. White soft paraffin, hard paraffin and squalane may be combined at a blend ratio of 70 to 90% by weight, 5 to 10% by weight and 5 to 20% by weight, respectively. A preferable preparation example contains Compound A, white soft paraffin, 5 ± 2% by weight of hard paraffin and 10 ± 2% by weight of squalane.

The pharmaceutical composition of the present invention may be orally or parenterally (e.g., locally, rectally, intravenously, etc.) administered to a mammal except a human being (e.g., a mouse, a rat, a hamster, a guinea pig, a rabbit, a cat, a dog, a pig, a cow, a horse, sheep, a monkey, etc.) as well as to a human being. A dosage amount depends on subjects, diseases, symptoms, dosages forms, administration routes, etc., and is usually, for example, in the range from about 0.01 mg to1 g per day in terms of the content of Compound A as an active ingredient. The dose may be administered at a time or in several divided doses.

A topical agent can be applied, for example, by application,inunction or spraying depending on the dosage form, etc. An application amount of the topical agent to the affected area can be selected depending on the content of the active ingredient, etc., and the topical agent can be applied, for example, at a time or in several divided amounts per day. The preferable application is once daily or twice daily.

The phrase "JAK" refers to one or more enzymes of JAK1, JAK2, JAK3, and TYK2 belonging to JAK family.

The phrase "inhibitJAK" refers to inhibiting functions of JAK to disappear or attenuate its activity, and inhibiting one or more enzymes belonging to JAK family.

The phrase "inhibit JAK" refers to preferably "inhibit human JAK". The inhibition of functions or the disappearance or attenuation of the activity is conducted preferably in the situations of human clinical application.

The "JAK inhibitor" may be any substances which inhibit JAK, and may be, for example, low-molecular weight compounds, nucleic acid, polypeptide, protein, antibody, vaccine, etc. The "JAK inhibitor" is preferably a "human JAK inhibitor".

The JAK inhibitor is preferably Compound A or a pharmaceutically acceptable salt thereof, and more preferably Compound A.

The term treatment used herein includes amelioration of a symptom, prevention of an aggravation, maintenance of a remission, prevention of an exacerbation, and prevention of a recurrence. The term prevention refers to suppressing occurrence of a symptom.

The term treatment may also include the delaying of the progression of the disease, disorder or condition, the amelioration, alleviation or relief of symptoms and complications, and/or cure or elimination of the disease, disorder or condition.
The term treatment may also mean the management and care of a patient for the purpose of combating the disease, condition or disorder.

The terms disease, disorder and condition as used herein are used interchangeably to specify a state of a patient which is not the normal physiological state of a human being.

Also disclosed is a method for treating or preventing alopecia areata, characterized by administering to a mammal a therapeutically effective amount of 3-[(3S,4R)-3-methyl-6-(7H-pyrrolo[2,3-d]pyrimidin-4-yl)-1,6-diazaspiro-[3.4]octan-1-yl]-3-oxopropanenitrile.

Also disclosed is a pharmaceutical composition for treating or preventing alopecia areata, comprising 3-[(3S,4R)-3-methyl-6-(7H-pyrrolo[2,3-d]pyrimidin-4-yl)-1,6-diazaspiro[3.4]octan-1-yl]-3-oxopropanenitrile and a pharmaceutically acceptable carrier.

Also disclosed is a use of 3-[(3S,4R)-3-methyl-6-(7H-pyrrolo[2,3-d]pyrimidin-4-yl)-1,6-diazaspiro[3.4]octan-1-yl]-3-oxopropanenitrile for treating or preventing alopecia areata.

The mammal is a human being, a mouse, a rat, a hamster, a guinea pig, a rabbit, a cat, a dog, a pig, a cow, a horse, sheep, a monkey, etc., and is preferably a human being. The human being is more preferably a person suffering from a disease who is in need of medical care.

The present invention is preferably a therapeutic or preventive agent for use in treating alopecia areata, comprising Compound A.

The present invention is preferably a pharmaceutical composition, comprising Compound A and a pharmaceutically acceptable carrier for use in treating alopecia areata.

### (B):

3-[(3S,4R)-3-methyl-6-(7H-pyrrolo[2,3-d]pyrimidin-4-yl)-1,6-diazaspiro[3,4]octan-1-yl]-3-oxopropanenitrile, Compound A, was described in previously in Patent Literature 1 and 2 as a JAK kinase inhibitor. As one of the aspects, the present invention describes its effect in topical treatment of Alopecia. The compound may be formulated in an ointment containing the active compound in 30 mg/g, white soft paraffin, hard paraffin and squalane. The treatment of alopecia may be at least twice daily applications. The duration of the treatment may be at least 12 weeks.

The invention thus provides the following aspects:
1. 3-[(3S,4R)-3-methyl-6-(7H-pyrrolo[2,3-d]pyrimidin-4-yl)-1,6-diazaspiro[3,4]-octan-1-yl]-3-oxopropanenitrile for use in the treatment of alopecia areata.
2. The compound according to the above for use in the topical treatment of alopecia areata
3. The compound for use according to any of the above aspects administered as a twice daily application for 12 weeks.
4. The compound for use according to any of the above aspects administered in an ointment.
5. The compound for use according to any of the above aspects administered as 30 mg/g concentration.
6. The compound for use according to any of the above aspects administered as 10 mg/g concentration.
7. The compound for use according to any of the above aspects administered as 5 mg/g concentration.

In any of the aspects of the invention according to the aspects above, the invention also provides:
the compound for use according to claim 1 having -an application area size of 200cm² to 700cm² or 30% to 100% scalp area.

In any of the aspects of the invention according to the aspects above, the invention also provides:
a dosing range 1.7mg/cm² of Compound A.

In embodiments of the invention and relating to any of the above aspects, the following embodiments of the invention are preferred:
In a preferred embodiment there is a change in SALT score from Visit 2 (Day 1, baseline) to Visit 6 (12 weeks, End of Treatment).

In another preferred embodiment the Safety profile by reported AE's. Exposure and standard safety data are acceptable.

In other embodiments the following parameters are improved (independently or together):
- Absolute SALT score
- SALT score change from baseline
- SALT score percentage change from baseline
- The event of having as at least 50% reduction in SALT score at Visit 6 (12 weeks, End of Treatment) compared to baseline (Day 1, baseline).
- Hair length
- Hair growth rate
- Relative hair thickness
- Hair type (velus hair, terminal hair)
- Hair color
- Global assessment of hair regrowth based on standardized photographs
- Subject global assessment of hair regrowth (SGA)
- Derivation of endpoints from the Alopecia Areata *Quality of Life*
- Derivation of endpoints from the Treatment Satisfaction Questionnaire Alopecia areata is the most common cause of inflammation-induced hair loss. It is a T-cell mediated autoimmune hair loss disease commonly resulting in bald patches on the scalp. The disease has an unpredictable course and there is currently no cure or satisfactory therapeutic options resulting in long-term remission. Alopecia areata has a significant negative impact on quality of life and self-esteem and there is a large unmet medical need for new effective treatment options. In the United States alopecia areata affects approximately 4.5 million people and the incidence rate is estimated to 0.1%-0.2%. The clinical presentation of hair loss is variable. The most common presentation is alopecia areata (90%) with one or more bald circumscribed patches on the scalp. Typically the affected skin has no sign of inflammation. Short fragile hairs (so-called exclamation point) are often seen in the periphery of lesions. Approximately 7% of patients may progress to alopecia totalis with a total loss of scalp hair or alopecia universalis with hair loss on the scalp and body. A few patients experience a diffuse type of alopecia areata or preferential loss of pigmented hair. Nail changes are seen in 20% of patients. Spontaneous regrowth of hair may occur in 50% of patients within one year and particularly in mild cases. Current evidence-based therapy is limited to topical (including injection) or systemic corticosteroids and sensitizing agents such as diphenylcyclopropenone and dinitrochlorobenzene. However, long-term use is often unacceptable and with disappointing efficacy. There is no evidence of efficacy for systemic immunosuppressants such as methotrexate, mycophenolate mofitile, cyclosporine A or azathioprine. Nor is there robust evidence for topical tacrolimus, cryotherapy or ultraviolet light A combined with oral psoralens (PUVA). Due to the lack of effective treatments and often devastating impact on patients' quality of life, many patients are willing to risk significant adverse drug effects and attempt off-label use of various treatments despite the lack of evidence supporting satisfactory outcomes or safety.

Also disclosed is a clinical trial wherein the claimed compound A is used in accordance with the present invention, to compare efficacy of twice daily topical ointment with 30 mg/g of Compound A, with ointment vehicle for 12 weeks in the treatment of hair loss in subjects with alopecia areata. The invention provides a safe treatment of alopecia areata using the above treatment.

As measure of hair growth, the following parameters has been defined:
- hair regrowth time course by SALT score (absolute, absolute change, percentage change) during treatment (for details on SALT score see below)
- hair regrowth by global assessment during treatment using standardized photographs and independent expert review.
- hair length, growth rate, relative thickness, hair type and color during treatment. And as subjective measures also:
- Subject Global Assessment (SGA) of hair regrowth during treatment
- Alopecia Areata Quality of Life Questionnaire at baseline and End of Treatment
- Treatment Satisfaction Questionnaire for Medication at End of Treatment
- systemic pharmacokinetics at steady state (4 weeks of treatment).

In a preferred embodiment there is a change in SALT score from Visit 2 (Day 1, baseline) to Visit 6 (12 weeks, End of Treatment).

In another preferred embodiment the Safety profile by reported AE's. Exposure and standard safety data are acceptable.

In other embodiments the following parameters are improved:
- Absolute SALT score
- SALT score change from baseline
- SALT score percentage change from baseline
- The event of having as at least 50% reduction in SALT score at Visit 6 (12 weeks, End of Treatment) compared to baseline (Day 1, baseline).
- Hair length
- Hair growth rate
- Relative hair thickness
- Hair type (velus hair, terminal hair)
- Hair color
- Global assessment of hair regrowth based on standardized photographs
- Subject global assessment of hair regrowth (SGA)
- Derivation of endpoints from the Alopecia Areata *Quality of Life*
- Derivation of endpoints from the Treatment Satisfaction Questionnaire

### EXAM PLES

### Example 1

Experiment 1. Promotion of hair growth by Compound A in C3H/HeJ mouse alopecia model.

An effect of promoting hair growth by Compound A was evaluated using a C3H/HeJ mouse alopecia model (Non-Patent Literature 1).

A skin specimen having a diameter of 2 to 2.5 cm was prepared from a retired C3H/HeJJcl mouse (CLEA Japan, Inc.) which had spontaneously developed alopecia universalis, and was grafted into a dorsal region of each of eight-week-old female C3H/HeJJcl mice (CLEA Japan, Inc.). Twenty weeks after the surgery, mice which developed alopecia throughout the whole bodies thereof were selected, and a carrier, 1% by weight of a test substance, 3% by weight of the test substance or a control substance was applied to the dorsal region at a volume of 50 µL once a day for 78 days. Day 1 was a day on which the application started. The carrier used was a mixture which contained white soft paraffin, hard paraffin and squalane at a blend ratio of 85 : 5 : 10. Compound A was used as the test substance. A betamethasone valerate ointment (0.12%) was used as the control substance.

The Hair Growth Index (HGI) in the dorsal region of each of the mice was determined on day 1, day 7, day 15, day 29, day 43, day 57, day 71 and day 79. The HGI for each of the mice was calculated by measuring, with naked eyes, the level of the state of hair growth(score; 0: no hair, 1: stubble, short broken hairs, 2: sparse intermediate length hairs, 3: normal length and density hairs) and the percentage (%) of the skin area in which each level of state of hair growth occurred,determining the product of the level of the state of hair growth (score) with the percentage (%) of the skin area in which its level of the state of hair growth occurred, and then summing all of the values of the products.

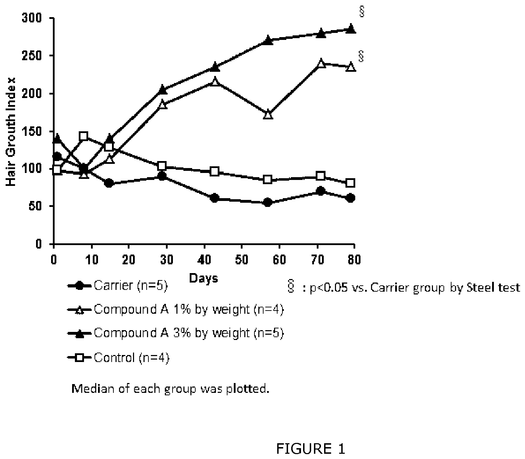

The results are shown in Fig. 1.

### Example 2

### Experiment 2. Promotion of hair growth by Compound A in C3H/HeJ mouse alopecia model

An effect of promoting hair growth by Compound A was evaluated using a C3H/HeJ mouse alopecia model (Non-Patent Literature 1).

A skin specimen having a diameter of 2 cm was prepared from a retired C3H/HeJJcl mouse (CLEA Japan, Inc.) which had spontaneously developed alopecia universalis, and was grafted into a dorsal region of each of eight-week-old female C3H/HeJJcl mice (CLEA Japan, Inc.). Twenty to twenty-two weeks after the surgery, mice which developed alopecia throughout the whole bodies thereof were selected, and a carrier, 0.3% by weight of a test substance, 3% by weight of the test substance or a control substance was applied to the dorsal region at a volume of 50 µL once a day for 84 days. Day 1 was a day on which the application started. The carrier used was a mixture which contained white soft paraffin, hard paraffin and squalane at a blend ratio of 85 : 5 : 10. Compound A was used as the test substance. A betamethasone valerate ointment (0.12%) was used as the control substance.

The Hair Growth Index (HGI) in the dorsal region of each of the mice was determined on day 1, day 19, day 29, day 43, day 57, day 71 and day 85. The HGI for each of the mice was calculated by measuring, with naked eyes, the level of the state of hair growth (score; 0: no hair, 1: stubble, short broken hairs, 2: sparse intermediate length hairs, 3: normal length and density hairs) and the percentage (%) of the skin area in which each level of state of hair growth occurred, determining the product of the level of the state of hair growth (score) with the percentage (%) of the skin area in which its level of the state of hair growth occurred, and then summing all of the values of the products.

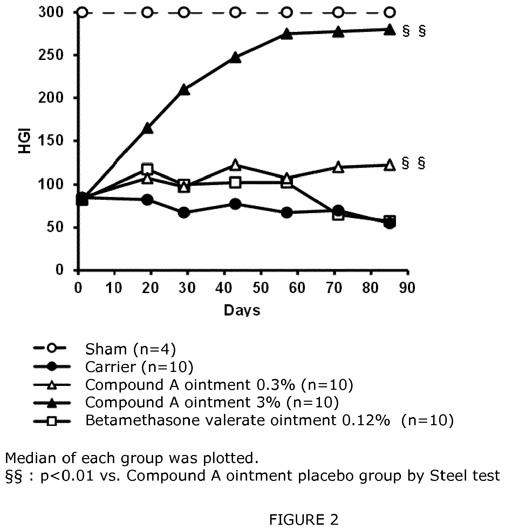

The results are shown in Fig. 2.

### Example 3

### Clinical trial:

This clinical trial is a phase 2, two-centre, prospective, randomized, double-blind, 2 arms, vehicle controlled, parallel group design with twice daily topical administration of Compound A in an ointment 30mg/g and Compound A ointment vehicle for 12 weeks to subjects with alopecia areata.

A total of 35 eligible subjects with alopecia areata affecting at least 30% of scalp area (SALT score ≥ = 30) at Visit1 (Screening) will be randomized in a 2:1 ratio to active treatment with Compound A ointment 30 mg/g twice daily and Compound A ointment vehicle twice daily Respectively. Maximally 30% of subjects enrolled must be classified as alopecia totalis or universalis.

The clinical trial is divided in to a Screening Phase (4 week duration), Treatment Phase (12 week duration) and Follow-up Phase (12 weeks).

Subjects will be screened at Visit 1(up to 28 days prior to Visit 2 (Day 1, baseline). Efficacy assessments will be performed at Visit 2, Visit 4 (4 weeks), Visit 5 (8 weeks), and Visit 6 (12 weeks, End of Treatment).Safety assessments will be performed at Visit 2, Visit 3 (2 weeks), Visit 4 (4 weeks), Visit 5 (8 weeks), Visit 6 (12 weeks, End of Treatment), and Visit 7 (2 week follow up, End of Trial) and by Phone Visit at Day 3.

For exploratory assessments skin punch biopsies and questionnaires will be performed at Visit 2 (Day 1, baseline) and Visit 6 (12 weeks, End of Treatment).

### Treatment Phase: (Day 1 to week 12)

At Visit 2 (Day 1, baseline) the subject's eligibility and informed consent will be rechecked and assessments will be conducted for baseline recording. For subjects who prior to randomization are found to be ineligible for participation in (screening failures) will have an End of Trial Form completed. Subjects will be randomized and instructed in the administration of Investigational product (IP).

Tubes with IP will be weighed before and after use. Subjects are given a diary to document IP administration and compliance. Subjects will have skin Biopsy 1 and Biopsy 2 taken from scalp.

On Day 3 a Phone Visit is scheduled to inquire about application site reactions, adverse events (AEs) and to address any questions regarding use of the IP. In case of any application site reactions or AE related or possibly related to the IP, or otherwise considered necessary by the investigator, an unscheduled site visit is arranged for an evaluation. In case the subject mentions an unrelated AE (e.g. knee pain) this will be documented and reported as an AE along with concomitant medication.

At Visit 3 (2 week) the biopsy wounds will be checked and the sutures removed. In addition safety will be assessed and recorded.

At Visit 4 (4 weeks), Visit 5 (8 weeks) and Visit 6 (12 weeks End of Treatment) efficacy and safety assessments will be recorded. At Visit 6 the subjects will also have skin Biopsy 3 taken from scalp. Compliance is checked by weighing returned IP and inspection of subject treatment diary. New IP and diary is administered except at Visit 6 (12 weeks, End of Treatment).

### Follow-up Phase: (2 weeks follow up, End of Trial).

At Visit 7 (2 week follow up, End of Trial) the biopsy wound from Visit 6 (12 weeks) will be checked and the sutures removed. In addition AEs and concomitant medication will be assessed and recorded. Subjects will have the End of Trial Form Completed. An unscheduled visit will be planned if there are ongoing non-serious AEs at the subjects End of Trial (Visit 7).

### Sample Size

In total 35 subjects will be randomized in the trial. It is estimated from clinical experience of the investigator that approximately 15% will withdraw before End of Treatment, resulting in 30 subjects evaluable for the primary endpoint. Screening failure (i.e. signed informed consent but not randomized) is estimated to 20 % resulting in a need to screen 44 subjects. Subjects withdrawing from the trial will not be replaced.

### Randomization

Eligible subjects who have signed the ICF will be assigned to treatment in accordance with a randomization schedule in a 2:1 ratio to either Compound A ointment 30mg/g or Compound A ointment vehicle.

In order to ensure the 2:1 ratio for subjects classified as AT or AU a stratified randomization will be used, stratifying by alopecia areata that are classified as neither AT nor AU (i.e. patch type) versus alopecia areata classified as AT or AU. The stratum of subjects classified as AT or AU may represent maximally 30% of the subjects randomized.

### Blinding

The trial is conducted as a double-blind trial. Neither site staff nor the subject will know the identity of the IP assigned to ensure the trial assessments are carried out without bias. The packaging and labeling of the IPs will contain no evidence of their identity.

### Subject Eligibility

The (sub) investigator should only randomise subjects who meet all eligibility criteria and are not put at undue risk by participating in the trial and can be expected to comply with the protocol. The subject's eligibility for the clinical trial must be checked according to the inclusion and exclusion criteria at Visit 1 (Screening) and Visit 2 (Day 1, baseline).

### Inclusion Criteria

All the following criteria must be met for inclusion of a subject in this clinical trial:
1. Subjects must have signed and dated informed consent after receiving verbal and written information about the clinical trial.
2. Male and female subjects between 18 and 65 years of age.
3. Subjects with unequivocal clinical diagnosis of moderate to severe scalp alopecia areata (patch type, totalis, universalis), as determined by the (sub) investigator, affecting a minimum of 30% scalp area at Visit 1 (Screening) and Visit 2 (Day 1, baseline).
4. Minimum 6 month duration of hair loss at Visit 1 (Screening). No upper limit time limit.
5. No evidence of significant ongoing hair loss or regrowth at Visit 1 or Visit 2.
6. Female subjects must be of either:
   a. Non-childbearing potential, post-menopausal (for at least 1 year), or have a confirmed clinical history of sterility (e.g. the subject is without a uterus) or,
   b. Childbearing potential (incl. women less that post-menopausal for 1 year), provided there is a confirmed negative urine pregnancy test prior to exposure, to rule out pregnancy.

FEMALE subjects of childbearing potential (and their male partners) and MALE subjects (and their female partner of childbearing potential) must be willing to use highly effective methods of contraception (Pearl index < 1%) to avoid pregnancy from enrolment and until 1 week after the last administration of IP. Adequate methods of contraception for female and male are defined:
1. Combined (estrogen and progestogen containing) hormonal contraception (oral, intravaginal transdermal) associated with inhibition of ovulation for at least one menstrual cycle prior to randomization (Visit 2).
2. Intrauterine device (IUD) for at least one menstrual cycle prior to randomization (Visit 2).
3. Intrauterine hormone-releasing system (IUS) for at least one menstrual cycle prior to randomization (Visit 2).
4. Bilateral tubal occlusion.
5. Vasectomized or vasectomized partner (partner should be sole partner for the subject.
6. Sexual abstinence (when this is in line with the preferred lifestyle of the subject).
7. In overall good health including well controlled diseases (e.g. hypertension, diabetes and thyroid disease) as determined by medical history, physical examination, electrocardiogram (ECG), vital signs (blood pressure, heart rate and body temperature) and clinical laboratory evaluation.
8. Subject must accept to not cut hair in the treated scalp areas during the trial.

### Exclusion Criteria

1. Females who are pregnant or are breast feeding.
2. Current signs of spontaneous hair regrowth.
3. Diffuse type alopecia areata.
4. Co-existing moderate to severe androgenic alopecia (Norwood-Hamilton stage IV-VI and Ludwig stage II and III.
5. Subjects with changed or expected changes in medication for thyroid disease within 6 month before Visit 1 (screening) or during the trial.
6. Systemic treatment with immunosuppressive drugs (e.g. methotrexate, cyclosporine, azathioprine), chloroquin derivatives, corticosteroids, or any other systemic therapy that in the opinion of the investigator could affect hair regrowth, within 6 weeks prior to randomization (inhaled or intra-nasal steroids corresponding to up to 1 mg prednisone for asthma or rhinitis may be used).
7. Biologics within 5 half-lives and minimum 4 weeks prior to randomization.
8. Systemic JAK inhibitor Ruxolitinib (Jakafi®/Jakavi®), Tofacitinib (Xeljanz®) at any time prior to randomization.
9. PUVA, UVB therapy, Exim-laser and other light based therapies within 4 weeks prior to randomization.
10. Topical immunotherapy (including diphenylcycloprophenone and dinitrochlorobenzene), anthrain, dithranol, or intralesional corticosteroids within 4 weeks prior to randomization.
11. Topical treatment with pimecrolimus, tacrolimus, calcipotriol, corticosteroids from WHO groups I to IV, minoxidil, alternative remedies incl. herbal extracts or any topical therapy which in the opinion of the investigator may affect hair regrowth on the scalp within 2 weeks prior to randomization.
12. Use of topical or systemic antibiotics within 2 weeks prior to randomization.
13. Known or suspected hypersensitivity to component(s) of the investigational product.
14. Known or suspected severe renal insufficiency or severe hepatic disorders.
15. Subjects with history of cancer except for basal cell carcinoma.
16. Subjects with history of an immunocompromising disease (e.g., lymphoma, HIV, Wiskott-Aldrich Syndrome).
17. A marked abnormal ECG at Visit 1 (screening), i.e. PR interval > 220 ms, QRS interval > 110 ms or corrected QT interval (Bazett) QTcB > 470 ms (female), 450 ms (male).
18. Supine BP at Visit 1 (screening) > 140/90 mmHg or < 90/50 mmHg after up to 15 min of rest.
19. Supine heart rate at Visit 1(screening) > 100 beats per minute (bpm) or < 50 bpm after up to 15 min of rest.
20. Body temperature (ear, tympanic) at Visit 1 (screening) >37.5c or <35.5c
21. Known hepatic dysfunction or hepatic dysfunction tested at screening (e.g. alanine aminotransferase [ALT], aspartate aminotransferase [AST], and gamma glutamyl transpeptidase [GGT] > 2 times the upper limit of normal.
22. History of concurrent diseases that could interfere with study evaluations or pose a safety concern for the subject.
23. Current participation in any other interventional clinical trial.
24. Subjects who have received treatment with any non-marketed drug substance (i.e. an agent which has not yet been made available for clinical use following registration) within 4 weeks prior to randomization or 5 half-lives whatever is the longest.
25. Previously randomized in this study.
26. Subjects known or, in the opinion of the investigator, is unlikely to comply with the Clinical Trial Protocol (e.g. alcoholism, drug dependency or psychotic state).
27. Subject with any contraindication to skin biopsy procedures: e.g., allergy to local anesthetics, topical antiseptics, bleeding tendency, treatment with anticoagulant drugs, history of poor would healing, and history of vasovagal hypotension or syncope (only applicable for subjects participating in optional skin biopsy sampling procedure).
28. Any abnormal physical, vital, laboratory or ECG finding that is clinically significant and would impact the safety of the subjects or the interpretation of the study results, as determined by the investigator's judgment.

### Restrictions during Trial

The use of drugs, defined as disallowed in the exclusion criteria is not permitted during the trial. Concomitant treatment for conditions other than alopecia areata may be continued throughout the trial without any change in dosage whenever possible.

### Method for SALT scores calculation.

Scalp area % of total scalp Assessment of % of area with alopecia areata Weighted % of area
Left side 18% (A1) 0-100% (B1) A1^{∗} B1=C1
Right side 18% (A2) 0-100% (B2) A2^{∗} B2=C2
Neck 24% (A3) 0-100% (B3) A3^{∗} B3=C3
Vertex 40% (A4) 0-100% (B4) A4^{∗} B4=C4
SALT Score ∑ C(1-4) = SALT SCORE

### Global assessment of overall hair regrowth

Global assessment of overall hair regrowth is evaluated by independent expert panel review of standardized photographs taken by the (sub-)investigator at visits . The subjects hair regrowth is categorized based on simultaneous evaluation of photographs from Visit 2 (baseline) and the photographs from the subsequent visits. The degree of hair regrowth is categorized and scored as detailed below.

### Global assessment of overall hair regrowth compared to baseline

### Regrowth categories Score recored in eCRF

Much worse -3
Moderately worse -2
Slightly worse -1
Unchanged 0
Slightly improved +1
Moderately improved +2
Much improved +3

### Hair length, thickness and color

At Visit 2 (Day 1, baseline) and Visit 4(4 weeks), Visit 5 (8 weeks), Visit 6 (12 weeks, End of Treatment) the investigator will assess for signs of hair regrowth by dermatoscopic evaluation (10x magnification). The investigator will select a representative treated area for evaluation of average hair length (mm), hair thickness relative to the subject's normal hair, type of hair, hair color and relative color to the subject's normal scalp hair color.

For subjects with alopecia totalis and universalis relative assessments are not applicable. If hair length should exceed what can be measure by dermatoscope a standard ruler is used. The hair growth rate will be calculated based on the time interval between assessments

### Investigator assessment of regrowth hair

Hair length from scalp in mm
Hair growth rate Change in length per day in mm/day
Thickness of regrowth hair Relative thickness to subjects normal scalp hair:
Much thinner, slightly thinner, same thickness, thicker Hair type Velus hair, Terminal hair
Color of regrowth hair: Absolute color Black, brown, red, blond, gray, white.
Relative color to subjects normal scalp hair:
   Much lighter, slightly lighter, same color, darker.

### Subject Assessments:

### Subject's global assessment of hair regrowth from baseline

At Visit 4 (4 weeks), Visit 5 (8 weeks) and Visit 6 (12 weeks, End of Treatment) the subject will be asked to evaluate the degree of hair regrowth that has occurred from Visit 2 (Day 1, baseline) with a Subject Global Assessment (SGA). The (sub)-investigator will explain the categories and scores of hair regrowth and the subject will state which category to record in eCRF. The subject will assess the hair regrowth before the investigator

### Subjects Global Assessment of hair regrowth:

### Score Regrowth categories

0 no regrowth
1 <25%of regrowth
2 25%-49% of regrowth
3 50%-74% of regrowth
4 75%-99% of regrowth
5 100% of regrowth

### Patient Reported Outcomes

There are no generally accepted tools for assessment of patient reported outcomes in subjects with alopecia areata. In this clinical trial the validated Alopecia Areata Quality of Life Questionnaire AAQLI) will be used at Visit 2 (Day 1, baseline) and Visit 6 (12 weeks, End of Treatment). The subjects Treatment Satisfaction Questionnaire for Medication (TSQM II) will be used at Visit 6 (12 weeks, End of Treatment). In addition an exploratory questionnaire (LEO questionnaire) has been developed by LEO to further address the patient behavior and preferences.

### Investigational product (IP):

**Composition of Compound A ointment 30mg/g (suspension of micronized Compound A):**

| Component | Amount (mg/g) |
|---|---|
| Compound A | 30 |
| Paraffin, white soft | 820 |
| Paraffin, hard | 50 |
| Squalane | 100 |

**Composition of Compound A ointment Vehicle:**

| Component | Amount (mg/g) |
|---|---|
| Sunset Yellow FCF aluminium lake (color added to the vehicle to match the color of Compound A ointment 30mg/g) | 0.041 |
| Paraffin, white soft | 850 |
| Paraffin, hard | 50 |
| Squalane | 100 |

### Administration

Route of administration: Topical (cutaneous).

Area of IP application: Areas on the scalp with hair loss (alopecia areata) at Visit 2 (Day 1) and approximately 1 cm (3/8 inch) into the surrounding area without hair loss. If hair regrowth is observed in the treated area the treatment continues until End of Treatment. If new areas of hair loss develops during the trial these are also treated.

Application area size: 200cm² to 700cm² (equivalent to 30% to 100% scalp area)

Dosing range mg/cm²: Thin layer covering the entire area corresponding to 1.7mg/cm² of Compound A ointment 30mg/g or Compound A ointment Dosing frequency Twice daily application for 12 weeks
Absorption time: The subject should allow 15 minutes for absorption of the ointment before applying wig, hairpieces or potential occluding material.

Time of day for dosing: Morning and evening (optimally 12 hours apart. Minimum 6 hours apart).

Daily total maximum: IP [mg] 2380mg Compound A ointment

Daily total maximum: API 71.4mg Compound A.

### CLAUSES

Also disclosed are the following clauses which as such are not part of the disclosure of the present invention:
Clause 1. A therapeutic or preventive agent for alopecia areata, comprising a compound represented by the following chemical structural formula: 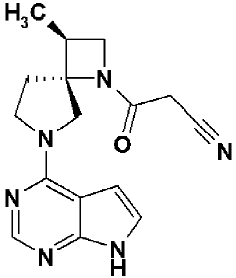 or a pharmaceutically acceptable salt thereof as an active ingredient.
Clause 2. The therapeutic or preventive agent for alopecia areata according to clause 1, wherein the therapeutic or preventive agent for alopecia areata is a topical agent.
Clause 3. The therapeutic or preventive agent for alopecia areata according to clause 2, wherein the topical agent is an ointment.
Clause 4. The therapeutic or preventive agent for alopecia areata according to clause 3, wherein the concentration of 3-[(3S,4R)-3-methyl-6-(7H-pyrrolo[2,3-d]pyrimidin-4-yl)-1,6-diazaspiro[3.4]octan-1-yl]-3-oxopropanenitrile in the ointment of clause 3 is 0.3% by weight.
Clause 5. The therapeutic or preventive agent for alopecia areata according to clause 3, wherein the concentration of 3-[(3S,4R)-3-methyl-6-(7H-pyrrolo[2,3-d]pyrimidin-4-yl)-1,6-diazaspiro[3.4]octan-1-yl]-3-oxopropanenitrile in the ointment of clause 3 is 1% by weight.
Clause 6. The therapeutic or preventive agent for alopecia areata according to clause 3, wherein the concentration of 3-[(3S,4R)-3-methyl-6-(7H-pyrrolo[2,3-d]pyrimidin-4-yl)-1,6-diazaspiro[3.4]octan-1-yl]-3-oxopropanenitrile in the ointment of clause 3 is 3% by weight.
Clause 7. The therapeutic or preventive agent for alopecia areata according to clause 1-3, wherein 3-[(3S,4R)-3-methyl-6-(7H-pyrrolo[2,3-d]pyrimidin-4-yl)-1,6-diazaspiro[3.4]octan-1-yl]-3-oxopropanenitrile is administered as 30 mg/g concentration.
Clause 8. The therapeutic or preventive agent for alopecia areata according to clause 1-3, wherein 3-[(3S,4R)-3-methyl-6-(7H-pyrrolo[2,3-d]pyrimidin-4-yl)-1,6-diazaspiro[3.4]octan-1-yl]-3-oxopropanenitrile is administered as 10 mg/g concentration.
Clause 9. The therapeutic or preventive agent for alopecia areata according to clause 1-3, wherein 3-[(3S,4R)-3-methyl-6-(7H-pyrrolo[2,3-d]pyrimidin-4-yl)-1,6-diazaspiro[3.4]octan-1-yl]-3-oxopropanenitrile is administered as 5 mg/g concentration.
Clause 10. The therapeutic or preventive agent for alopecia areata according to clause 1-9, wherein 3-[(3S,4R)-3-methyl-6-(7H-pyrrolo[2,3-d]pyrimidin-4-yl)-1,6-diazaspiro[3.4]octan-1-yl]-3-oxopropanenitrile is administered as a twice daily application.
Clause 11. The therapeutic or preventive agent for alopecia areata according to clause 10, wherein 3-[(3S,4R)-3-methyl-6-(7H-pyrrolo[2,3-d]pyrimidin-4-yl)-1,6-diazaspiro[3.4]octan-1-yl]-3-oxopropanenitrile is administered as a twice daily application for 12 weeks.
Clause 12. The therapeutic or preventive agent for alopecia areata according to clause 1-11, wherein 3-[(3S,4R)-3-methyl-6-(7H-pyrrolo[2,3-d]pyrimidin-4-yl)-1,6-diazaspiro[3.4]octan-1-yl]-3-oxopropanenitrile is administered as a twice daily application in an area of 200 cm² to 700 cm².
Clause 13. The therapeutic or preventive agent for alopecia areata according to clause 1-12, wherein 3-[(3S,4R)-3-methyl-6-(7H-pyrrolo[2,3-d]pyrimidin-4-yl)-1,6-diazaspiro[3.4]octan-1-yl]-3-oxopropanenitrile is administered as a twice daily application in an amount of 1.7 mg/cm².
Clause 14. A pharmaceutical composition for use in the treatment of alopecia areata, comprising a compound represented by the following chemical structural formula: 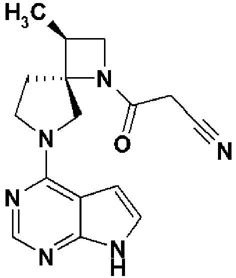 or a pharmaceutically acceptable salt thereof, together with a pharmaceutically acceptable carrier(s).
Clause 15. The pharmaceutical composition according to clause 14, wherein the pharmaceutical composition is a topical agent.
Clause 16. The pharmaceutical composition according to clause 14 or 15,wherein the pharmaceutical composition is an ointment.
Clause 17. The pharmaceutical composition according to clause 16, wherein the concentration of 3-[(3S,4R)-3-methyl-6-(7H-pyrrolo[2,3-d]pyrimidin-4-yl)-1,6-diazaspiro[3.4]octan-1-yl]-3-oxopropanenitrile is 0.3% by weight.
Clause 18. The pharmaceutical composition according to clause 16, wherein the concentration of 3-[(3S,4R)-3-methyl-6-(7H-pyrrolo[2,3-d]pyrimidin-4-yl)-1,6-diazaspiro[3.4]octan-1-yl]-3-oxopropanenitrile is 1% by weight.
Clause 19. The pharmaceutical composition according to clause 16, wherein the concentration of 3-[(3S,4R)-3-methyl-6-(7H-pyrrolo[2,3-d]pyrimidin-4-yl)-1,6-diazaspiro[3.4]octan-1-yl]-3-oxopropanenitrile is 3% by weight.
Clause 20. The pharmaceutical composition according to any one of the clauses 14 - 16 wherein 3-[(3S,4R)-3-methyl-6-(7H-pyrrolo[2,3-d]pyrimidin-4-yl)-1,6-diazaspiro-[3,4]octan-1-yl]-3-oxopropanenitrile is administered as 30 mg/g concentration.
Clause 21. The pharmaceutical composition according to any one of the clauses 14 - 16 wherein 3-[(3S,4R)-3-methyl-6-(7H-pyrrolo[2,3-d]pyrimidin-4-yl)-1,6-diazaspiro-[3,4]octan-1-yl]-3-oxopropanenitrile is administered as 10 mg/g concentration.
Clause 22. The pharmaceutical composition according to any one of the clauses 14 - 16 wherein 3-[(3S,4R)-3-methyl-6-(7H-pyrrolo[2,3-d]pyrimidin-4-yl)-1,6-diazaspiro-[3,4]octan-1-yl]-3-oxopropanenitrile is administered as 5 mg/g concentration.
Clause 23. The pharmaceutical composition according to any one of the clauses 14 - 22 wherein 3-[(3S,4R)-3-methyl-6-(7H-pyrrolo[2,3-d]pyrimidin-4-yl)-1,6-diazaspiro-[3,4]octan-1-yl]-3-oxopropanenitrile is administered as a twice daily application.
Clause 24. The pharmaceutical composition according to clause 23 wherein wherein 3-[(3S,4R)-3-methyl-6-(7H-pyrrolo[2,3-d]pyrimidin-4-yl)-1,6-diazaspiro[3,4]octan-1-yl]-3-oxopropanenitrile is administered as a twice daily application for 12 weeks.
Clause 25. The pharmaceutical composition according to any one of the clauses 14 - 24 wherein 3-[(3S,4R)-3-methyl-6-(7H-pyrrolo[2,3-d]pyrimidin-4-yl)-1,6-diazaspiro-[3,4]octan-1-yl]-3-oxopropanenitrile is administered in an area of 200 cm² to 700 cm².
Clause 26. The pharmaceutical composition according to any one of the clauses 14 - 25 wherein 3-[(3S,4R)-3-methyl-6-(7H-pyrrolo[2,3-d]pyrimidin-4-yl)-1,6-diazaspiro-[3,4]octan-1-yl]-3-oxopropanenitrile is administered in an amount of 1.7 mg/cm².
Clause 27. 3-[(3S,4R)-3-methyl-6-(7H-pyrrolo[2,3-d]pyrimidin-4-yl)-1,6-diazaspiro-[3,4]octan-1-yl]-3-oxopropanenitrile for use in the treatment of alopecia areata.
Clause 28. The compound according to clause 27 for use in the topical treatment of alopecia areata.
Clause 29. The compound according to clause 27 or 28 administered as a twice daily application.
Clause 30. The compound according to clause 29 administered as a twice daily application for 12 weeks.
Clause 31. The compound according to any one of the clauses 27 - 30 administered in an ointment.
Clause 32. The compound according to clause 31, wherein the concentration of 3-[(3S,4R)-3-methyl-6-(7H-pyrrolo[2,3-d]pyrimidin-4-yl)-1,6-diazaspiro[3.4]octan-1-yl]-3-oxopropanenitrile is 0.3% by weight.
Clause 33. The compound according to clause 31, wherein the concentration of 3-[(3S,4R)-3-methyl-6-(7H-pyrrolo[2,3-d]pyrimidin-4-yl)-1,6-diazaspiro[3.4]octan-1-yl]-3-oxopropanenitrile is 1% by weight.
Clause 34. The compound according to clause 31, wherein the concentration of 3-[(3S,4R)-3-methyl-6-(7H-pyrrolo[2,3-d]pyrimidin-4-yl)-1,6-diazaspiro[3.4]octan-1-yl]-3-oxopropanenitrile is 3% by weight.
Clause 35. The compound according to any one of the clauses 27 - 31 administered as 30 mg/g concentration.
Clause 36. The compound according to any one of the clauses 27 - 31 administered as 10 mg/g concentration.
Clause 37. The compound according to any one of the clauses 27 - 31 administered as 5 mg/g concentration.
Clause 38. The compound according to any one of the clauses 27 - 37 wherein the compound is administered in an area of 200 cm² to 700 cm².
Clause 39. The compound according to any one of the clauses 27 - 38 wherein the compound is administered in an amount of 1.7 mg/cm².
Clause 40. A use of 3-[(3S,4R)-3-methyl-6-(7H-pyrrolo[2,3-d]pyrimidin-4-yl)-1,6-diazaspiro[3,4]octan-1-yl]-3-oxopropanenitrile, in the manufacture of a pharmaceutical composition for the treatment of alopecia areata.
Clause 41. The use according to clause 40 wherein the pharmaceutical composition is a topical pharmaceutical composition.
Clause 42. The use according to clause 41 wherein the pharmaceutical composition is an ointment.
Clause 43. The use according clause 42 wherein the concentration of 3-[(3S,4R)-3-methyl-6-(7H-pyrrolo[2,3-d]pyrimidin-4-yl)-1,6-diazaspiro[3,4]octan-1-yl]-3-oxopropanenitrile is 0.3% by weight.
Clause 44. The use according clause 42 wherein the concentration of 3-[(3S,4R)-3-methyl-6-(7H-pyrrolo[2,3-d]pyrimidin-4-yl)-1,6-diazaspiro[3,4]octan-1-yl]-3-oxopropanenitrile is 1% by weight.
Clause 45. The use according to clause 42 wherein the concentration of 3-[(3S,4R)-3-methyl-6-(7H-pyrrolo[2,3-d]pyrimidin-4-yl)-1,6-diazaspiro[3,4]octan-1-yl]-3-oxopropanenitrile is 3% by weight.
Clause 46. The use according to any one of the clauses 40 - 42 wherein 3-[(3S,4R)-3-methyl-6-(7H-pyrrolo[2,3-d]pyrimidin-4-yl)-1,6-diazaspiro[3,4]octan-1-yl]-3-oxopropanenitrile is administered as 30 mg/g concentration.
Clause 47. The use according to any one of the clauses 40 - 42 wherein 3-[(3S,4R)-3-methyl-6-(7H-pyrrolo[2,3-d]pyrimidin-4-yl)-1,6-diazaspiro[3,4]octan-1-yl]-3-oxopropanenitrile is administered as 10 mg/g concentration.
Clause 48. The use according to any one of the clauses 40 - 42 wherein 3-[(3S,4R)-3-methyl-6-(7H-pyrrolo[2,3-d]pyrimidin-4-yl)-1,6-diazaspiro[3,4]octan-1-yl]-3-oxopropanenitrile is administered as 5 mg/g concentration.
Clause 49. The use according to any one of the clauses 40 - 48 wherein 3-[(3S,4R)-3-methyl-6-(7H-pyrrolo[2,3-d]pyrimidin-4-yl)-1,6-diazaspiro[3,4]octan-1-yl]-3-oxopropanenitrile is administered as a twice daily application.
Clause 50. The use according to clause 49 wherein 3-[(3S,4R)-3-methyl-6-(7H-pyrrolo[2,3-d]pyrimidin-4-yl)-1,6-diazaspiro[3,4]octan-1-yl]-3-oxopropanenitrile is administered as a twice daily application for 12 weeks.
Clause 51. The use according to any one of the clauses 40 - 50 wherein 3-[(3S,4R)-3-methyl-6-(7H-pyrrolo[2,3-d]pyrimidin-4-yl)-1,6-diazaspiro[3,4]octan-1-yl]-3-oxopropanenitrile is administered in an area of 200 cm² to 700 cm².
Clause 52. The use according to any one of the clauses 40 -51 wherein 3-[(3S,4R)-3-methyl-6-(7H-pyrrolo[2,3-d]pyrimidin-4-yl)-1,6-diazaspiro[3,4]octan-1-yl]-3-oxopropanenitrile is administered in an amount of 1.7 mg/cm².
Clause 53. A method for treating alopecia areata in a subject in need thereof, which method comprises the step of administering to said subject a therapeutically effective amount of 3-[(3S,4R)-3-methyl-6-(7H-pyrrolo[2,3-d]pyrimidin-4-yl)-1,6-diazaspiro-[3,4]octan-1-yl]-3-oxopropanenitrile.
Clause 54. The method according to clause 53 wherein the administration is topical.
Clause 55. The method according to clause 54 wherein the topical administration is an ointment.
Clause 56. The method according to clause 55 wherein the concentration of 3-[(3S,4R)-3-methyl-6-(7H-pyrrolo[2,3-d]pyrimidin-4-yl)-1,6-diazaspiro[3,4]octan-1-yl]-3-oxopropanenitrile is 0.3% by weight.
Clause 57. The method according to clause 55 wherein the concentration of 3-[(3S,4R)-3-methyl-6-(7H-pyrrolo[2,3-d]pyrimidin-4-yl)-1,6-diazaspiro[3,4]octan-1-yl]-3-oxopropanenitrile is 1% by weight.
Clause 58. The method according to clause 55 wherein the concentration of 3-[(3S,4R)-3-methyl-6-(7H-pyrrolo[2,3-d]pyrimidin-4-yl)-1,6-diazaspiro[3,4]octan-1-yl]-3-oxopropanenitrile is 3% by weight.
Clause 59. The method according to any one of the clauses 53 - 55 wherein 3-[(3S,4R)-3-methyl-6-(7H-pyrrolo[2,3-d]pyrimidin-4-yl)-1,6-diazaspiro[3,4]octan-1-yl]-3-oxopropanenitrile, is administered in a concentration of 30 mg/g.
Clause 60. The method according to any one of the clauses 53 - 55 wherein 3-[(3S,4R)-3-methyl-6-(7H-pyrrolo[2,3-d]pyrimidin-4-yl)-1,6-diazaspiro[3,4]octan-1-yl]-3-oxopropanenitrile, is administered in a concentration of 10 mg/g.
Clause 61. The method according to any one of the clauses 53 - 55 wherein 3-[(3S,4R)-3-methyl-6-(7H-pyrrolo[2,3-d]pyrimidin-4-yl)-1,6-diazaspiro[3,4]octan-1-yl]-3-oxopropanenitrile, is administered in a concentration of 5 mg/g.
Clause 62. The method according to any one of the clauses 53 - 61 wherein 3-[(3S,4R)-3-methyl-6-(7H-pyrrolo[2,3-d]pyrimidin-4-yl)-1,6-diazaspiro[3,4]octan-1-yl]-3-oxopropanenitrile is administered as a twice daily application.
Clause 63. The method according to clause 62 wherein 3-[(3S,4R)-3-methyl-6-(7H-pyrrolo[2,3-d]pyrimidin-4-yl)-1,6-diazaspiro[3,4]octan-1-yl]-3-oxopropanenitrile is administered as a twice daily application for 12 weeks.
Clause 64. The method according to any one of the clauses 53 - 63 wherein 3-[(3S,4R)-3-methyl-6-(7H-pyrrolo[2,3-d]pyrimidin-4-yl)-1,6-diazaspiro[3,4]octan-1-yl]-3-oxopropanenitrile is administered in an area of 200 cm² to 700 cm².
Clause 65. The method according to any one of the clauses 56 - 64 wherein 3-[(3S,4R)-3-methyl-6-(7H-pyrrolo[2,3-d]pyrimidin-4-yl)-1,6-diazaspiro[3,4]octan-1-yl]-3-oxopropanenitrile is administered in an amount of 1.7 mg/cm².

### INDUSTRIAL APPLICABILITY

The present invention provides a new pharmaceutical use of 3-[(3S,4R)-3-methyl-6-(7H-pyrrolo[2,3-d]pyrimidin-4-yl)-1,6-diazaspiro[3.4]octan-1-yl]-3-oxopropanenitrile for alopecia areata as a target disease.

## Claims

1. A compound, 3-[(3S,4R)-3-methyl-6-(7H-pyrrolo[2,3-d]pyrimidin-4-yl)-1,6-diazaspiro[3,4]octan-1-yl]-3-oxopropanenitrile or a pharmaceutically acceptable salt thereof, for use in the treatment of alopecia areata.

2. The compound for use according to claim 1 wherein the treatment is topical.

3. The compound for use according to claim 1 or 2 wherein the compound is administered in an ointment.

4. The compound for use according to claim 3 wherein the concentration of the compound in the ointment is 0.3% by weight.

5. The compound for use according to claim 3 wherein the concentration of the compound in the ointment is 1% by weight.

6. The compound for use according to claim 3 wherein the concentration of the compound in the ointment is 3% by weight.

7. The compound for use according to any of the one of the preceding claims wherein the compound is administered in an amount of 1.7 mg/cm².

8. The compound for use according to any of the one of the preceding claims wherein the compound is administered as a twice daily application.

9. The compound for use according to claim 8, wherein the compound is administered as a twice daily application for 12 weeks.

10. A therapeutic or preventive agent for use in the treatment of alopecia areata, comprising a compound represented by the following chemical structural formula: 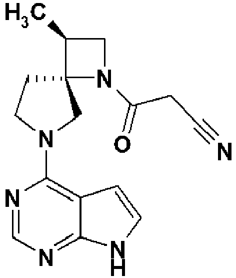 or a pharmaceutically acceptable salt thereof, as an active ingredient.

11. The therapeutic or preventive agent for use in the treatment of alopecia areata according to claim 10, wherein the pharmaceutical composition is a topical agent.

12. The therapeutic or preventive agent for use in the treatment of alopecia areata according to claim 11,wherein the topical agent is an ointment.

13. The therapeutic or preventive agent for use in the treatment of alopecia areata according to claim 12, wherein the concentration of 3-[(3S,4R)-3-methyl-6-(7H-pyrrolo[2,3-d]pyrimidin-4-yl)-1,6-diazaspiro[3.4]octan-1-yl]-3-oxopropanenitrile is 0.3% by weight.

14. The therapeutic or preventive agent for use in the treatment of alopecia areata according to claim 12, wherein the concentration of 3-[(3S,4R)-3-methyl-6-(7H-pyrrolo[2,3-d]pyrimidin-4-yl)-1,6-diazaspiro[3.4]octan-1-yl]-3-oxopropanenitrile is 1% by weight.

15. The therapeutic or preventive agent for use in the treatment of alopecia areata according to claim 12, wherein the concentration of 3-[(3S,4R)-3-methyl-6-(7H-pyrrolo[2,3-d]pyrimidin-4-yl)-1,6-diazaspiro[3.4]octan-1-yl]-3-oxopropanenitrile is 3% by weight.

16. A pharmaceutical composition for use in the treatment of alopecia areata, comprising 3-[(3S,4R)-3-methyl-6-(7H-pyrrolo[2,3-d]pyrimidin-4-yl)-1,6-diazaspiro-[3,4]octan-1-yl]-3-oxopropanenitrile, or a pharmaceutically acceptable salt thereof, together with a pharmaceutically acceptable carrier(s).

## Patentansprüche

1. Verbindung, 3-[(3S,4R)-3-Methyl-6-(7H-pyrrolo[2,3-d]pyrimidin-4-yl)-1,6-diazaspiro[3,4]octan-1-yl]-3-oxo-propannitril, oder ein pharmazeutisch unbedenkliches Salz davon zur Verwendung bei der Behandlung von Alopecia areata.

2. Verbindung zur Verwendung nach Anspruch 1, wobei die Behandlung topisch ist.

3. Verbindung zur Verwendung nach Anspruch 1 oder 2, wobei die Verbindung in einer Salbe verabreicht wird.

4. Verbindung zur Verwendung nach Anspruch 3, wobei die Konzentration der Verbindung in der Salbe 0,3 Gew.-% entspricht.

5. Verbindung zur Verwendung nach Anspruch 3, wobei die Konzentration der Verbindung in der Salbe 1 Gew.-% entspricht.

6. Verbindung zur Verwendung nach Anspruch 3, wobei die Konzentration der Verbindung in der Salbe 3 Gew.-% entspricht.

7. Verbindung zur Verwendung nach einem der vorhergehenden Ansprüche, wobei die Verbindung in einer Menge von 1,7 mg/cm² verabreicht wird.

8. Verbindung zur Verwendung nach einem des einen der vorhergehenden Ansprüche, wobei die Verbindung als eine Anwendung zweimal täglich verabreicht wird.

9. Verbindung zur Verwendung nach Anspruch 8, wobei die Verbindung als eine Anwendung zweimal täglich 12 Wochen lang verabreicht wird.

10. Therapeutikum oder vorbeugendes Mittel zur Verwendung bei der Behandlung von Alopecia areata, umfassend eine Verbindung, die durch die folgende chemische Strukturformel dargestellt wird: 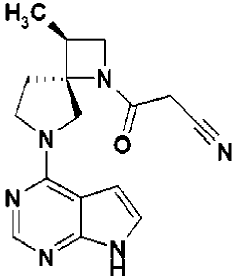 oder ein pharmazeutisch unbedenkliches Salz davon als ein Wirkstoff.

11. Therapeutikum oder vorbeugendes Mittel zur Verwendung bei der Behandlung von Alopecia areata nach Anspruch 10, wobei die pharmazeutische Zusammensetzung ein topisches Mittel ist.

12. Therapeutikum oder vorbeugendes Mittel zur Verwendung bei der Behandlung von Alopecia areata nach Anspruch 11, wobei das topische Mittel eine Salbe ist.

13. Therapeutikum oder vorbeugendes Mittel zur Verwendung bei der Behandlung von Alopecia areata nach Anspruch 12, wobei die Konzentration von 3-[(3S,4R)-3-Methyl-6-(7H-pyrrolo[2,3-d]pyrimidin-4-yl)-1,6-diazaspiro[3,4]octan-1-yl]-3-oxo-propannitril 0,3 Gew.-% entspricht.

14. Therapeutikum oder vorbeugendes Mittel zur Verwendung bei der Behandlung von Alopecia areata nach Anspruch 12, wobei die Konzentration von 3-[(3S,4R)-3-Methyl-6-(7H-pyrrolo[2,3-d]pyrimidin-4-yl)-1,6-diazaspiro[3,4]octan-1-yl]-3-oxo-propannitril 1 Gew.-% entspricht.

15. Therapeutikum oder vorbeugendes Mittel zur Verwendung bei der Behandlung von Alopecia areata nach Anspruch 12, wobei die Konzentration von 3-[(3S,4R)-3-Methyl-6-(7H-pyrrolo[2,3-d]pyrimidin-4-yl)-1,6-diazaspiro[3,4]octan-1-yl]-3-oxo-propannitril 3 Gew.-% entspricht.

16. Pharmazeutische Zusammensetzung zur Verwendung bei der Behandlung von Alopecia areata, umfassend 3-[(3S,4R)-3-Methyl-6-(7H-pyrrolo[2,3-d]pyrimidin-4-yl)-1,6-diazaspiro[3,4]octan-1-yl]-3-oxo-propannitril, oder ein pharmazeutisch unbedenkliches Salz davon, zusammen mit (einem) pharmazeutisch unbedenklichen Träger(n).

## Revendications

1. Composé, le 3-[(3S,4R)-3-méthyl-6-(7H-pyrrolo[2,3-d]pyrimidin-4-yl)-1,6-diazaspiro [3,4]octan-1-yl]-3-oxopropanénitrile ou un sel pharmaceutiquement acceptable de celui-ci, destiné à être utilisé dans le traitement de l'alopécie en aires.

2. Composé destiné à être utilisé selon la revendication 1 dans lequel le traitement est topique.

3. Composé destiné à être utilisé selon la revendication 1 ou la revendication 2 dans lequel le composé est administré dans une pommade.

4. Composé destiné à être utilisé selon la revendication 3 dans lequel la concentration du composé dans la pommade est de 0,3 % en poids.

5. Composé destiné à être utilisé selon la revendication 3 dans lequel la concentration du composé dans la pommade est de 1 % en poids.

6. Composé destiné à être utilisé selon la revendication 3 dans lequel la concentration du composé dans la pommade est de 3 % en poids.

7. Composé destiné à être utilisé selon l'une quelconque des revendications précédentes dans lequel le composé est administré en une quantité de 1,7 mg/cm².

8. Composé destiné à être utilisé selon l'une quelconque des revendications précédentes dans lequel le composé est administré à deux applications par jour.

9. Composé destiné à être utilisé selon la revendication 8, dans lequel le composé est administré à deux applications par jour pendant 12 semaines.

10. Agent thérapeutique ou de prévention destiné à être utilisé dans le traitement de l'alopécie en aires, comprenant un composé représenté par la formule structurelle chimique suivante : 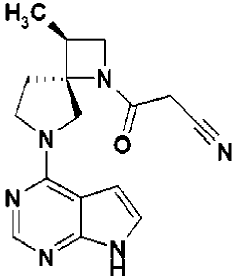 ou un sel pharmaceutiquement acceptable de celui-ci, en tant que principe actif.

11. Agent thérapeutique ou de prévention destiné à être utilisé dans le traitement de l'alopécie en aires selon la revendication 10, dans lequel la composition pharmaceutique est un agent topique.

12. Agent thérapeutique ou de prévention destiné à être utilisé dans le traitement de l'alopécie en aires selon la revendication 11, dans lequel l'agent topique est une pommade.

13. Agent thérapeutique ou de prévention destiné à être utilisé dans le traitement de l'alopécie en aires selon la revendication 12, dans lequel la concentration du 3-[(3S,4R)-3-méthyl-6-(7H-pyrrolo[2,3-d]pyrimidin-4-yl)-1,6-diazaspiro[3.4]octan-1-yl]-3-oxop ropanénitrile est de 0,3 % en poids.

14. Agent thérapeutique ou de prévention destiné à être utilisé dans le traitement de l'alopécie en aires selon la revendication 12, dans lequel la concentration du 3-[(3S,4R)-3-méthyl-6-(7H-pyrrolo[2,3-d]pyrimidin-4-yl)-1,6-diazaspiro[3.4]octan-1-yl]-3-oxop ropanénitrile est de 1 % en poids.

15. Agent thérapeutique ou de prévention destiné à être utilisé dans le traitement de l'alopécie en aires selon la revendication 12, dans lequel la concentration du 3-[(3S,4R)-3-méthyl-6-(7H-pyrrolo[2,3-d]pyrimidin-4-yl)-1,6-diazaspiro[3.4]octan-1-yl]-3-oxop ropanénitrile est de 3 % en poids.

16. Composition pharmaceutique destinée à être utilisée dans le traitement de l'alopécie en aires, comprenant le 3-[(3S,4R)-3-méthyl-6-(7H-pyrrolo[2,3-d]pyrimidin-4-yl)-1,6-diazaspiro-[3,4]octan-1-yl]-3-oxo propanénitrile, ou un sel pharmaceutiquement acceptable de celui-ci, ensemble avec un ou plusieurs excipients pharmaceutiquement acceptables.
